# EUROPEAN PATENT SPECIFICATION

(11) **EP 1 806 983 B1**
(45) Date of publication and mention of the grant of the patent: **14.05.2008**
(21) Application number: 05799655.5
(22) Date of filing: 28.10.2005
(51) Int. Cl.: A23L 1/305, A23K 1/00, A61K 31/198, A61K 38/05, A61K 38/06, A61P 1/00

(54) **NUTRITIONAL AND/OR PHARMACEUTICAL PREPARATION FOR USE IN PROPHYLAXIS AND TREATMENT OF DISTURBANCES IN MICROELEMENTS ABSORPTION FROM THE ALIMENTARY CANAL**
NÄHRMITTEL UND/ODER PHARMAZEUTISCHES PRÄPARAT FÜR DIE PROPHYLAXE UND BEHANDLUNG VON STÖRUNGEN DER SPURENELEMENTABSORPTION AUS DEM VERDAUUNGSTRAKT
PREPARATION NUTRITIVE ET/OU PHARMACEUTIQUE DESTINÉE A ETRE UTLISÉE EN PROPHYLAXIE ET TRAITEMENT DE TROUBLES DANS L' ABSORPTION DE MICRO-ELEMENTS A PARTIR DU TUBE DIGESTIF

(30) Priority: 29.10.2004 PL 37093704
(43) Date of publication of application: 18.07.2007
(73) Proprietor: SGP & SONS AB, 223 52 Lund (SE); Pierzynowski, Stefan G., 223 52 Lund (SE); Pierzynowski, Lukasz, 02784 Warszawa (PL); Pierzynowski, Kacper, 02784 Warszawa (PL)
(72) Inventor: SGP & SONS AB, 223 52 Lund (SE); Pierzynowski, Stefan G., 223 52 Lund (SE); Pierzynowski, Lukasz, 02784 Warszawa (PL); Pierzynowski, Kacper, 02784 Warszawa (PL)
(74) Representative: Akerman, Marten Lennart
(86) International application number: PCT/PL2005/000068
(87) International publication number: WO 2006/046880

(56) References cited:
- WO-A-03/043626
- WO-A-03/055508
- DATABASE WPI Section Ch, Week 200374 Derwent Publications Ltd., London, GB; Class B05, AN 2003-780074 XP002347419 & CN 1 153 634 A (KONG Y) 9 July 1997 (1997-07-09)
- DATABASE WPI Section Ch, Week 199402 Derwent Publications Ltd., London, GB; Class B04, AN 1994-010975 XP002364379 & JP 05 316999 A (AJINOMOTO CO INC) 3 December 1993 (1993-12-03)

## Description

An object of the invention is nutritional and/or pharmaceutical preparation for the use in the prophylaxis and treatment of disturbances in microelements absorption from the alimentary canal and the use of pharmaceutical preparation as a factor stimulating microelements absorption from the alimentary canal into the blood ensuring proper - physiological - curative level of these compounds in the blood in people and animals.

Alfa-keto-glutarate acid (AKG), glutamine derivative, is a key compound in Krebs cycle.

AKG, the mediate product in the main metabolic cycle, is transformed into glutaminic acid by the complex of glutamine dehydrogenase. The last is in turn transformed into glutamine in the presence of glutamine synthetase.

In the rapid process of izocitrate oxidative decarboxylation with the glutamate dehydrase stable and non-toxic AKG is generated:

Izocitrate + NAD⁺ → α - ketoglutarate + CO₂ + NADH

Alpha-ketoglutarate acid ( AKG ) is thought to be the adequate exogenous precursor of endogenously synthetized glutamine and through glutamate acid which is necessary to the synthesis of other amino acids with 5-carbons chain, such glutamine, arginine, proline, histidine. These amino acids are transformed into α-glutamate which then is oxidatively deaminated with the glutamate dehydrase. AKG originates in the effect of these reactions.

Beside the function as energy donor alpha-ketoglutarate acid (AKG) plays also the protective role for proteins not allowing to their catabolizm. He works as "sweeper" of ammonium ions. Alpha-amino groups (residues) are transformed into ammonium ions in the process of oxidative deamination of glutamate with the glutamate dehydrase. AKG plays an important role in the process of organism detoxification and in the retention of ammonium ion protecting the break-up of proteins. Glutamine, the main precursor of AKG in , the organism, is a main source of energy for the intestine epithelial cells. Its definite amount (concentration) is necessary to maintain the continuous rate of cells division necessary to keep main absorption functions.
The greatest part of necessary glutamine used by enterocytes originates directly in the intestine but food and blood are also its important source. In the case of glutamine deficiency in the alimentary canal its reserve is mobilized on the way of muscular tissue decomposition.
Many studies on animals and people with the intestine function disturbances and supporting treatment with glutamine were performed. Glutamine supplied to primarily starved and then fed intravenously rats had had the positive effect on the improving of the intestine wall structure and decreased bacterial translocation to the circulatory system. There was also observed that glutamine has regenerative properties in the damaged wall of intestine in people after radiation and cytotoxic treatment.
It is known that bacteriemia caused by the translocation of the intestinal bacterial flora is often observed after operations when the dysfunction of alimentary canal occures. That was proved that in such cases glutamine given *per os* or intravenously in animals and people has the protective activity minimalizing microbes dislocation. These observations leads to conclusion that in the metabolic stress the administration of glutamine as a therapeutic agent can have positive results.

WO 0304362 discloses mineral rich compositions for good mineral absorption comprising poly-gamma-glutamine acid having a molecular weight between 10.000 and 300.000.
However in practical practice glutamate is still difficult for using because is unstable in solution and poorly soluble.
To avoid these problems the interest of investigators has directed into glutamine precursors.
The nutritional or /and pharmaceutical preparation according to the invention distinguishes itself that contains alpha-keto-glutarate or/and glutamine or/and glutamate or/and ornithine of alpha-keto-glutarate, dipeptide of glutamine and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipeptides of glutamine or glutamate with other amino acid in a dose 0.01-0.5 g/kg/day and given orally stimulates the absorption of iron, zinc, coppers, manganese, strontium, calcium, phosphorus and other microelements from the alimentary canal to the blood ensuring their proper - physiological - curative level in the blood in people and animals.
It is profitable if the nutritional or/and pharmaceutical preparation is curative and preventive in some diseases, especially in anaemia, postpartum enterostasis, heart dysfunction, atherosclerosis, tetany, osteoporosis, arthritis, intestine dysfunction, muscular dysfunction, dementia, the diminution of immunity, increased rates of bacterial, viral and mycotic infections, increased morbidity of neoplastic and other diseases in people and animals and also increases the productiveness and the efficiency of farm animals as swine, poultry, sheep, cows, horses, goat and others.
Other nutritional or/and pharmaceutical preparation according to the invention distinguishes itself that contains iron (Fe) or other ions of bivalent metals being the alimentary microelements in a dose 0.0001-0.01 g/kg/day and increases the absorption into the blood and the metabolism in entereocytes of alpha-keto-glutarate or/and glutamine or/and glutamate or/and ornithine of alpha-keto-glutarate, dipeptides of glutamine and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipeptides of glutamine or glutamate with other amino acid ensuring their physiological - curative - preventive level in blood in people and animals.
It is profitable in other nutritional or/and pharmaceutical preparation if the nutritional or/and pharmaceutical preparation has therapeutic and preventive properties in some diseases, especially in anaemia, especially in anaemia, postpartum enterostasis, heart dysfunction, atherosclerosis, tetany, osteoporosis, arthritis, intestine dysfunction, muscular dysfunction, dementia, the diminution of immunity, increased rates of bacterial, viral and mycotic infections, increased morbidity for neoplastic and other diseases in people and animals and also increases the productiveness and the yield of farm animals as swine, poultry, sheeps, cows, horses, goats and others.
The use of the nutritional or/and pharmaceutical preparation according to the invention distinguishes itself that the preparation containing alpha-keto-glutarate or/and glutamine or/and glutamate or/and ornithine of alpha-keto-glutarate, dipeptide glutamines and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipeptide glutamines or glutamate with other amino acid in a dose 0.01-0.5 g/kg/day administered orally is used as a stimulator of absorbtion of iron, zinc, coppers, manganes, strontium, calcium, phosphorus and other microelements from the alimentary canal to the blood.
The first other use of the nutritional or/and pharmaceutical preparation, according to the invention, distinguishes itself that the preparation containing alpha-keto-glutarate or/and glutamine or/ang glutamate or/and ornithine of alpha-keto-glutarate, dipeptide glutamines and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipeptide glutamines or glutamate with other amino acid in a dose 0.01-0.5 g/kg/day is used to assure proper - physiological - curative level of these substances in the blood in people and animals having therapeutic and preventive properties in the following diseases: anaemia, postpartum enterostasis, heart dysfunction, atherosclerosis, tetany, osteoporosis, arthritis, intestine dysfunction, muscular dysfunction, dementia, the diminution of immunity, increased rates of bacterial, viral and mycotic infections, increased morbidity for neoplastic and other diseases in people and animals.
The second other use of the nutritional or/and pharmaceutical preparation, according to the invention, distinguishes itself that the preparation containing alpha-keto-glutarate or/and glutamine or/and glutamate or/and ornithine of alpha-keto-glutarate, dipeptide glutamines and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipeptide glutamines or glutamate with other amino acid in a dose 0.01-0.5 g/kg/day is used to increase the productiveness and yield of farm animals as the swine, poultry, sheeps, cows, horses, goats and others.
The third other use of the nutritional or/and pharmaceutical preparation, according to the invention, distinguishes itself-that the preparation containing alpha-keto-glutarate or/and glutamine or/and glutamate or/and ornithine of alpha-keto-glutarate, dipeptide glutamines and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipeptide glutamines or glutamate with other amino acid in a dose 0.01-0.5 g/kg/day is used to increase the absorption of manganese (Mn) to the bile and liver what makes possible the use of this preparation as a component of oral contrast used during examination and diagnosing of the neoplasms of the liver and other tissues of the alimentary canal in people and and animals.
The fourth other use of the nutritional or/and pharmaceutical preparation, according to the invention distinguishes itself the preparation containing iron (Fe) or other ions of bivalent metals being the alimentary microelements in a dose 0.0001-0.01 g/kg/day, increasing the absorption into blood and metabolism in entereocytes of alpha-keto-glutarate or/and glutamine or/and glutamate or/and ornithine of alpha-keto-glutarate, dipeptide glutamines and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipeptide glutamines or glutamate with other amino acid is used to assure their physiological - curative - preventive level in blood in the following diseases: anaemia, postpartum enterostasis, heart dysfunction, atherosclerosis, tetany, osteoporosis, arthritis, intestine dysfunction, muscular dysfunction, dementia, the diminution of immunity, increased rates of bacterial, viral and mycotic infections, increased morbidity for neoplastic and other diseases in people and animals.
The fifth other use of the nutritional or/and pharmaceutical preparation, according to the invention, distinguishes itself that contains iron (Fe) or other ions of bivalent metals being alimentary microelements in a dose 0.0001-0.01 g/kg/day and increases the absorption into blood and the metabolism in entereocytes of alpha-keto-glutarate or/and glutamine or/and glutamate or/and ornithine of alpha-keto-glutarate, dipeptide glutamines and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipeptide glutamines or glutamate with other amino acid is used for the protection and the increase of the productiveness and yield of such farm animals as swine, poultry, sheeps, cows, horses, goats and others.
In compliance with the invention the influence of AKG on the absorption of iron and other microelements was examined. Iron and the other microelements, for example zinc, copper, manganese are necessary to the synthesis of haemoglobin, mioglobine, cytochromes, and other enzymes. The malabsorption of iron and other microelements is a main cause of many diseases, for example anaemia, postpartum enterostasis, heart dysfunction, atherosclerosis, tetany, osteoporosis, arthritis, intestine dysfunction, muscular dysfunction, dementia, the diminution of immunity, increased rates of bacterial, viral and mycotic infections, increased morbidity for neoplastic diseases.
The influence of AKG as a component of intravenous nutrition was intensively examined in different patients' clinical condition. The intravenous nutrition formulas with the standard composition and with the addition of branch chain amino acids (BCAA), glutamine and AKG were investigated. That was proved that BCAA has no significance in the parenteral nutrition while glutamine and alfa-ketoglutarate administered parenterally increase the level of the constitutional glutamine in the muscles and decrease the negative nitrogen balance.
AKG reduces also the ammonia level what is essential in the medical care. High concentration of the ammonia in blood (ammonaemia) leads to hepatocirrhosis; however the most serious negative activity of this compound is its influence on the brain. Increased level of ammonia can lead to brain oedema and coma.
The invention is presented in examples on the base of performed examinations: Fig.1 - the diagram of the increased absorption of iron (Fe) from the ileum in pigs, Fig.2 - the increased absorption of iron (Fe) into blood after oral administration of AKG in people, and Fig.3 - increased absorption of zinc (Zn) into blood after oral administration of AKG in people, Fig.4 - increased absorption of manganese (Mn) (µmol/kg of body weight) from the intestine after enteral administration of AKG in rats.

### Example I.

### Animals

Investigations were performed on 3 piglets of the Swedish race Landrace weighting 30 - 35 kg. Animals originated from the university herd (Swedish Agricultural University, Dept. of Agricultural Biosystems and Technology, Lund). Young pigs were fed 2 times a day at the same time (9:00-10:00 a.m. and 3:00-4:00 p.m.) with a standard fodder (Vaxfor, Lantmanen, Sweden) with a daily dose 50g /kg body weight. They were watered *ad libitum*. Animals were held separately in boxes with automatically regulated 12 hour's periods of day and night.

### The surgical operation

Sterile catheters and T-fistulas used in the experience were made of silicone tubes with maintained medical standards (Dow Cornning).
Piglets were starved for 9-12 hours before the operation.
Before the operation animals received a sedative drug - azoperone (Stresnil, 2 mg/kg body weight). Then the inhalation induction of general anaesthesia was performed (Fluothan 0.5 - 1.5 vol %). Duodenal and pelvic fistulas were made in all animals and AKG infusion was administered. With the catheters inserted to the portal vein and the common carotid-artery the blood samples for later analyses were collected.

Two incisions were done: one 10-15 cm close to the right hunger fossa, second 2-3 cm between the brachial joint and the angle of the mandible. Duodenal fistula was placed behind the opening of the pancreatic duct to the duodenum. Pelvic T-fistula (int. diameter 3.18 mm and ext. diameter 4.64 mm - both fistulas) was introduced to the pelvic intestine at the end of the caeco-iliac ligament.
The catheter to the portal vein (int. diameter 0.64 mm and ext. diameter 1.19mm) was introduced through the liver and fixed with several sutures (0-2 Catgut , Ethicon, England).
The catheter placed in portal vein and both intestinal fistulas were brought out the body in the line of incision. After dissection of zygomatic vein the catheter was introduced in the cardiac direction on the depth of 7 cm. The vein was closed with the double ligature (0-3 silicone , Ethicon, England) and brought outside the body with the surgical needle in the dorsal part of the neck.
The abdomen and the neck wounds were closed with two layers of sutures: the peritoneum (only in the abdomen wound) and muscles were closed with twisted suture (0-0 Catgut , Ethicon), the skin was closed with the interrupted sutures (Suturamid 2-0, Ethicon).
After operation young pigs were placed in separate boxes. During 5 postoperative days animals were fed twice a day, thet received water *ad libitum.* The operation was performed by the skilled surgeon in accordance with surgical techniques.

### Infusions /doses:

solution 1 - 35.1 g AKG / I pH = 5.0 : 0.15 g/kg body weight
solution 2 - 5.64 g FeSO₄ / I pH = 2.0 : 10 mg/kg body weight
solution 3 - 35.1 g AKG / I, FeSO₄/ I pH = 2.0 : 0.15 g+10 mg/kg body weight
The above infusions, repeating twice, were administered to animals in two-days intervals according to the rule of "Latin square" (table 1)

**Table 1 . The plan of experience**

| | **Infusions** | | |
|---|---|---|---|
| **Day of experience** | **AKG** | **FeSO₄** | **AKG/FeSO₄** |
| 1 | 5d | 6d | 7d |
| 3 | 5b | 6b | 7b |
| 5 | 7d | 5d | 6d |
| 7 | 7b | 5b | 6b |
| 9 | 6d | 7d | 5d |
| 11 | 6b | 7b | 5b |

| | | | |
|---|---|---|---|
| d - infusion to the duodenum b - infusion to the pelvic intestine 5, 6, 7 - the number of animals | | | |

Before infusion animals were starved. The daily dose of food was given during evening feeding at 3:00 p.m.
All infusions were started at 9:00 a.m. Blood samples were taken 30 minutes before and 15, 30, 60 and 120 minutes after the infusion.

### 2. Infusions composition.

| | **studia** | | |
|---|---|---|---|
| | **solution 1** | **solution 2** | **solution 3** |
| pH | 5 | 2 | 2 |
| | mmol/l | mmol/l | mmol/l |
| AKG⁻⁻ | 240 | - | 240 |
| Na⁺ | 373 | 159 | 88 |
| K⁺ | 55 | - | 13 |
| Ca⁺⁺ | - | - | - |
| Mg⁺⁺ | - | - | - |
| H⁺ | 0.01 | 10 | 10 |
| Cl⁻ | - | 182.5 | - |
| Fe⁺⁺ | - | 37 | 37 |
| SO4₂⁻⁻ | - | 37 | 37 |

### Results

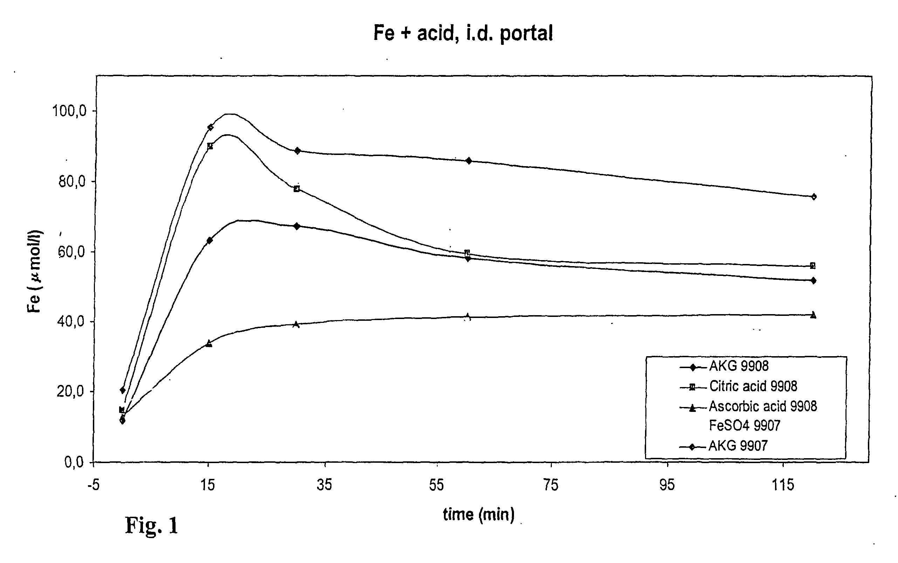

Results are presented on Figure 1 and Tables 3, 4.
AKG administered enterally significantly increases iron absorption from the intestine into the blood (Fig. 1, Table 3), FeSO₄ increases the absorption of AKG from the intestine into the blood (Table 4).

**Table 3**

| The iron concentration (µmol/l) in plasma of young pigs receiving FeSO4 and FeSO4/AKG infusions to the duodenum and the pelvic intestine (average ± SD, n = 3, observations = 6) | | | | |
|---|---|---|---|---|
| **FeSO4 infusion** | | | | |
| | To the duodenum | | To pelvic intestine | |
| Time (min) | Zygomatic vein | Portal vein | Zygomatic vein | Portal vein |
| -30 | 18,2 ± 1,53 ^{a} | 16,33 ±1,04 ^{a} 19,67 ± 7,97 ^{a} | | 19,17 ± 8,69 ^{a} |
| 15 | 29,17 ± 5.51 ^{b} | 28,5 ± 4,09 ^{b} 18,83 ± 6,81 ^{a} | | 18,00 ± 5,41 ^{a} |
| 30 | 39,50 ± 4,09 ^{c} | 37,67 ± 5,3 ^{cb} 18,83 ± 6,81 ^{a} | | 17,67 ± 6,53 ^{a} |
| 60 | 43,0 ± 4,82 ^{c} | 40,17 ± 4,62 ^{c} 18,00 ± 7,47 ^{a} | | 16,83 ± 6,81 ^{a} |
| 120 | 39.5 ± 3,5 ^{c} | 37,17 ± 4,37 ^{c} 15,67 ± 6,43 ^{a} | | 15,33 ± 7,18 ^{a} |

| | **FeSO4/AKG infusion** | | | |
|---|---|---|---|---|
| Time (min) | Zygomaticus vein | Portal vein | Zygomaticus vein | Portal vein |
| -30 | 20,33 ± 5,62 ^{a} | 20,50 ± 5,89 ^{a} | 20,47 ± 7,85 ^{a} | 20,00 ± 8,05 ^{a} |
| 15 | 98,83 ± 29,72 ^{d} | 95,50 ± 29,51 ^{d} | 36,27 ± 25,54 ^{bc} | 32,00 ± 24,89 ^{bc} |
| 30 | 96,00 ± 27,88 ^{d} | 88,67 ± 23,71 ^{d} | 36,93 ± 27,49 ^{bc} | 33,17 ± 27,76 ^{bc} |
| 60 | 93,17 ± 17,96 ^{d} | 85,83 ± 15,50 ^{d} | 34,30 ± 26,01 ^{bc} | 29,83 ± 22,21 ^{bc} |
| 120 | 80,50 ± 17,06 ^{d} | 75,67 ± 15,43 ^{d} | 27,67 ± 19,06 ^{bc} | 25,67 ± 18,45 ^{bc} |

**Table 4. AKG concentration (µg/l) in plasma of young pigs receiving FeS04 and FeSO4/AKG infusions to the duodenum and the pelvic intestine (average ± SD, n = 3, observations = 6)**

| | **AKG infusion** | | | |
|---|---|---|---|---|
| | To the duodenum | | To pelvic intestine | |
| Time (min) | Zygomatic vein | Portal vein | Zygomatic vein | Portal vein |
| -30 | 1,87±0,64 ^{a} | 3,38 ± 0,54^{b} | 1,47 ± 0,65 ^{a} | 3,80 ± 2,57 ^{b} |
| 15 | 11,32±6,94 ^{d} | 28,62 ± 2,84 ^{f} | 2,96 ± 0,29 ^{b} | 5,33 ± 2,48 ^{b} |
| 30 | 13,62±3,91^{d} | 22,86 ± 4,08 ^{f} | 2,55 ± 1,26 ^{b} | 4,06 ± 2,55 ^{b} |
| 60 | 7,72±2,69 ^{cb} | 13,53 ± 3,04 ^{e} | 2,58 ± 0,26 ^{b} | 4,18 ± 0,63 ^{b} |
| 120 | 4,82±3,39 ^{b} | 11,11 ± 6,70 ^{ce} | 2,60 ± 0,73 ^{b} | 4,05 ± 0,16 ^{b} |

| | **FeSO4/AKG infusion** | | | |
|---|---|---|---|---|
| | To the duodenum | | To pelvic intestine | |
| Time (min) | Zygomatic vein | Portal vein | Zygomatic vein | Portal vein |
| -30 | 3,06 ± 0,93 ^{b} | 4,78 ± 1,71 ^{b} | 3,44 ± 1,51 ^{b} | 4,53 ± 1,40 ^{b} |
| 15 | 28,53 ± 21,89 ^{f} | 47,80 ± 23,62 ^{g} | 13,99 ± 16,35 ^{def} | 15,96 ± 20,72 ^{def} |
| 30 | 17,80 ± 8,53 ^{c} | 27,41 ± 9,80 ^{f} | 9,67 ± 8,17 ^{bce} | 9,77 ± 10,60 ^{bce} |
| 60 | 9,62 ± 4,09 ^{c} | 16,60 ± 4,86 ^{c} | 3,43 ± 1,23 ^{b} | 5,27 ± 4,12 ^{bc} |
| 120 | 4,89 ± 5,03 ^{b} | 7,74 ± 1,77 ^{cb} | 4,89 ± 5,03 ^{b} | 6.51 ± 5,70 ^{bc} |

| | | | | |
|---|---|---|---|---|
| Letters indicate statistically significant differences (p<0.05). | | | | |

### Example II

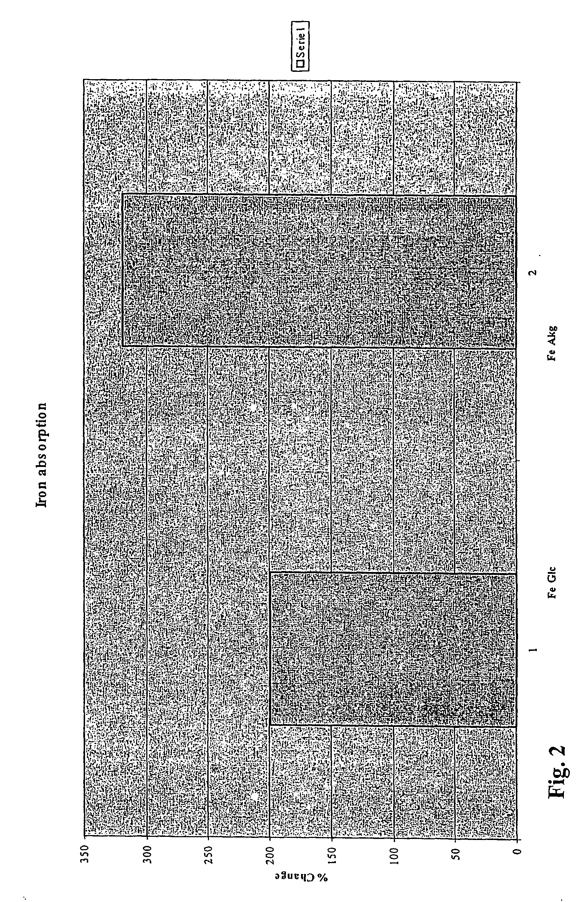

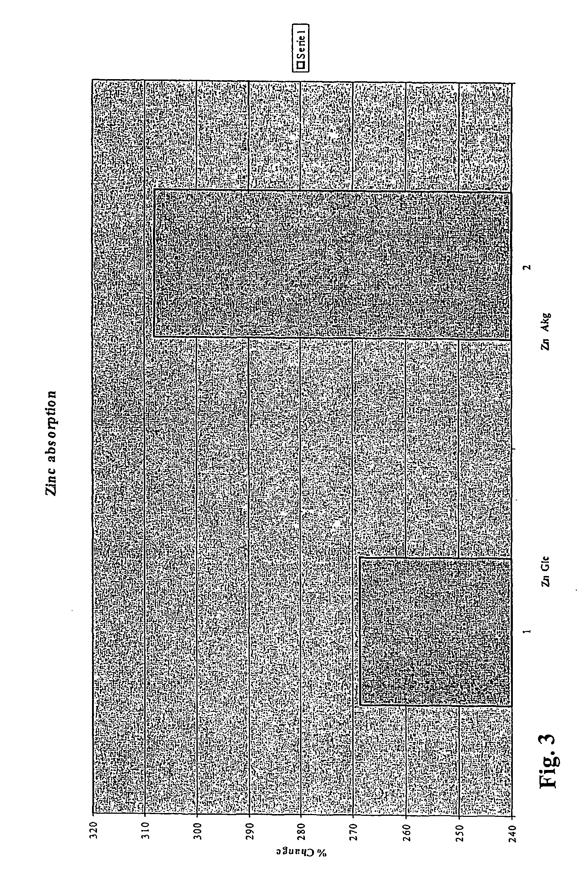

Research was performed on 4 volunteers.
At the first day, after randomization and after taking the initial blood samples, volunteers drank on an empty stomach 200 mg zinc sulphate - ZnS04 - dissolved in 200 ml of H₂O (Sovezink, Tika Läkemedel AB, Lund) and a tablet of iron formate - Fe(HCOO)₂ in a dose 200 mg (Erco-Fer, Orion). Immediately after the drugs consumption volunteers drank 1 litre of 10% glucose in 0.9% NaCl solution. Next blood samples were taken 30, 60 120 and 180 minutes after glucose drinking. Two days later these volunteers consumed identical doses of iron and zinc and drank thereafter 1 litre of 10% AKG-soda salt solution.
Results:Results are presented on Figures 2 and 3. AKG administered orally increases quantitative absorption of Fe2+ and Zn2+ ions from the alimentary canal into the blood in people.

### Example III

Experiments were performed on 24 rats not eating for night before the experience. The induction of general anaethesia was done with ketamine 50 mg/kg (Ketalar).
The catheters to the duodenum, the back part of jejunum and to the biliary duct were inserted in animals. The experience was performed simultaneously in 6 animals.
After 30-minutes period of post-operative stabilization 6 animals received MnCl₂ to the duodenum, next 6 received MnCl₂ to the back part of jejunum and next 6 animals received MnCl₂ with the addition of AKG-soda salt in a dose 0.5 g/kg body weight to the duodenum. The last group of 6 animals received MnCl₂ with AKG-soda salt in a dose 0.5 g/kg body weight to the back part of intestine.
The content of manganese in the bile collected 2 hours after the infusion and in the liver dissected directly after the end of the bile collection and after the euthanasia of the animal was examined.

### Results

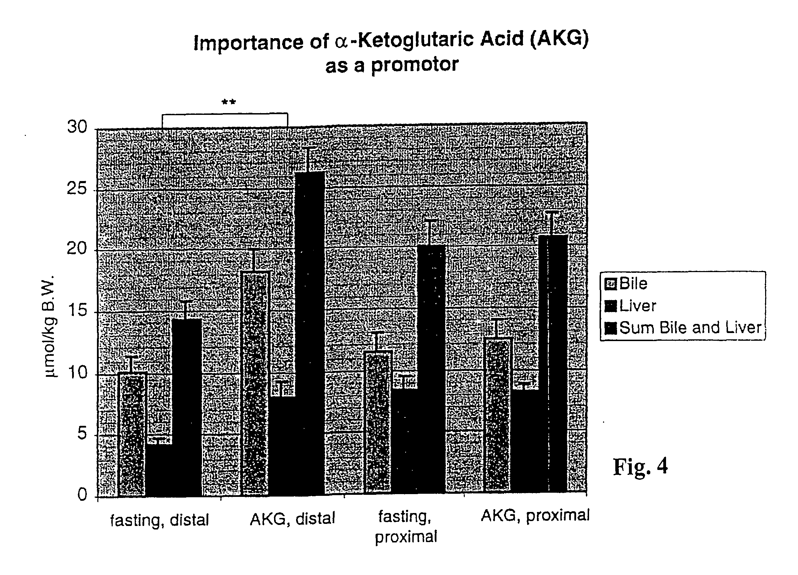

Results indicate that AKG increases the absorption of the manganese ( Mn ) from the back part of jejunum (Figure 4)

## Claims

1. Use of alpha-keto-glutarate or/and glutamine or/and glutamate or/and ornithine of alpha-keto-glutarate, dipeptide of glutamine and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipepetides of glutamine or glutamate with other amino acid for the manufacture of a nutritional and/or pharmaceutical preparation for use in the prophylaxis and/or the treatment of disturbances in the microelements absorption from the alimentary canal.

2. Use according to claim 1, wherein the preparation stimulates the absorption of iron, zinc, copper, manganese, strontium, calcium, phosphorus and other microelements from the alimentary canal to the blood ensuring proper - physiological - curative level of these microelements in the blood in people and animals.

3. Use according to any of claims 1-2, wherein the preparation is used to assure the proper - physiological - curative level of microelements in the blood in people and animals being curative and preventive in the following diseases: anaemia, postpartum enterostasis, heart dysfunction, atherosclerosis, tetany, osteoporosis, arthritis, intestine dysfunction, muscular dysfunction, dementia, the diminution of immunity, increased rates of bacterial, viral and mycotic infections, increased morbidity of neoplastic and other diseases in people and animals.

4. Use according to claim 1, wherein the preparation is used to increases the productiveness and the yield of farm animals as swine, poultry, sheep, cows, horses, goat and others.

5. Use according to any of claims 1-4, wherein the preparation is administered orally in a dose of 0.01-0.5 g/kg day.

6. Use according to any of claims 1-5, wherein the preparation further contains iron (Fe) ions or other ions of bivalent metals being alimentary microelements in a dose of 0.0001-0.01 g/kg/day.

7. Use of alpha-keto-glutarate or/and glutamine or/and glutamate or/and ornithine of alpha-keto-glutarate, dipeptide of glutamine and other amino acids or/and tripeptides of glutamine and other amino acids or/and glutamate with other amino acid or/and salts of alpha-keto-glutarate or/and glutamine or/and glutamate or/and ornithine of alpha-keto-glutarate, dipepetides of glutamine or glutamate with other amino acid for the manufacture of a diagnostic preparation to be used to increase the absorption of manganese (Mn) to the bile and liver permitting the use of this preparation as a component of the oral contrast used during examination and diagnosing of the neoplasms in the liver and other tissues of the alimentary canal in people and animals.

## Patentansprüche

1. Verwendung von alpha-Ketoglutarat oder/und Glutamin oder/und Glutamat oder/und Ornithin von alpha-Ketoglutarat, Dipeptiden von Glutamin und anderen Aminosäuren oder/und Tripeptiden von Glutamin und anderen Aminosäuren oder/und Glutamat mit anderen Aminosäuren oder/und Salzen von alpha-Ketoglutarat oder/und Glutamin oder/und Glutamat oder/und Ornithin von alpha-Ketoglutarat, Dipeptiden von Glutamin oder Glutamat mit anderen Aminosäuren für die Herstellung einer Nährmittel- und/oder pharmazeutischen Zusammensetzung zur Verwendung in der Prophylaxe und/oder Behandlung von Störungen in der Absorption von Mikroelementen aus dem Verdauungstrakt.

2. Verwendung nach Anspruch 1, wobei die Zusammensetzung die Absorption von Eisen, Zink, Kupfer, Mangan, Strontium, Calcium, Phosphor und anderen Mikroelementen aus dem Verdauungstrakt ins Blut stimuliert, um einen korrekten physiologischen-kurativen Spiegel dieser Mikroelemente im Blut von Menschen oder Tieren zu gewährleisten.

3. Verwendung nach einem der Ansprüche 1-2, wobei die Zusammensetzung verwendet wird, um einen korrekten physiologischen-kurativen Spiegel der Mikroelemente im Blut von Menschen und Tieren zu gewährleisten, wobei die folgenden Krankheiten geheilt oder ihnen vorgebeugt wird: Anämie, postnatale Enterostase, Herzdysfunktion, Atherosklerose, Tetanie, Osteoporose, Arthritis, Darmdysfunktion, Muskeldysfunktion, Demenz, Minderung der Immunität, gesteigerte Anzahl an bakteriellen, viralen und mykotischen Infektionen, gesteigerte Morbidität bei neoplastischen und anderen Erkrankungen bei Menschen und Tieren.

4. Verwendung nach Anspruch 1, wobei die Zusammensetzung verwendet wird, um die Produktivität und die Ausbeute von Nutztieren, wie Schweinen, Geflügel, Schafen, Kühen, Pferden, Ziegen und anderen zu steigern.

5. Verwendung nach einem der Ansprüche 1-4, wobei die Zusammensetzung in einer Dosis von 0,01-0,5 g/kg pro Tag oral verabreicht wird.

6. Verwendung nach einem der Ansprüche 1-5, wobei die Zusammensetzung ferner Eisen-(Fe) ionen oder andere Ionen von bivalenten Metallen, die Nahrungsmittel-Mikroelemente sind, in einer Dosis von 0,0001-0,01 g/kg pro Tag enthält.

7. Verwendung von alpha-Ketoglutarat oder/und Glutamin oder/und Glutamat oder/und Ornithin von alpha-Ketoglutarat, Dipeptiden von Glutamin und anderen Aminosäuren oder/und Tripeptiden von Glutamin und anderen Aminosäuren oder/und Glutamat mit anderen Aminosäuren oder/und Salzen von alpha-Ketoglutarat oder/und Glutamin oder/und Glutamat oder/und Ornithin von alpha-Ketoglutarat, Dipeptiden von Glutamin oder Glutamat mit anderen Aminosäuren für die Herstellung einer diagnostischen Zusammensetzung, die für eine Steigerung der Absorption von Mangan (Mn) in der Galle und Leber zu verwenden ist, was die Verwendung dieser Zusammensetzung als eine Komponente eines oralen Kontrastmittels während der Untersuchung und Diagnose von Neoplasmen in der Leber oder anderen Geweben des Verdauungstrakts in Menschen oder Tieren ermöglicht.

## Revendications

1. Utilisation d'un alpha-céto-glutarate ou/et d'une glutamine ou/et d'un glutamate ou/et d'une omithine d'alpha-céto-glutarate, d'un dipeptide de glutamine et d'autres acides aminés ou/et de tripeptides de glutamine et d'autres acides aminés ou/et d'un glutamate avec un autre acide aminé ou/et des sels d'alpha-céto-glutarate ou/et d'une glutamine ou/et d'un glutamate ou/et d'une omithine d'alpha-céto-glutarate, de dipeptides de glutamine ou de glutamate avec un autre acide aminé pour la fabrication d'une préparation nutritionnelle et/ou pharmaceutique pour une utilisation dans la prophylaxie et/ou le traitement de troubles de l'absorption de micro-éléments du tube digestif.

2. Utilisation selon la revendication 1, dans laquelle la préparation stimule l'absorption de fer, de zinc, de cuivre, de manganèse, de strontium, de calcium, de phosphore et d'autres micro-éléments du tube digestif vers le sang garantissant un niveau correct, physiologique, curatif de ces micro-éléments dans le sang chez les personnes et les animaux.

3. Utilisation selon l'une quelconque des revendications 1 et 2, dans laquelle la préparation est utilisée pour assurer un niveau correct, physiologique, curatif des micro-éléments dans le sang chez des personnes et des animaux et est curative et préventive dans les maladies suivantes : l'anémie, l'entérosténose postpartum, un dysfonctionnement cardiaque, l'athérosclérose, la tétanie, l'ostéoporose, l'arthrite, un dysfonctionnement de l'intestin, un dysfonctionnement musculaire, la démence, la diminution de l'immunité, des taux accrus d'infections bactériennes, virales et mycotiques, une morbidité accrue de néoplasme et d'autres maladies chez les personnes et les animaux.

4. Utilisation selon la revendication 1, dans laquelle la préparation est utilisée pour augmenter la productivité et le rendement d'animaux de la ferme tels que le porc, la volaille, les moutons, les vaches, les chevaux, les chèvres et autres.

5. Utilisation selon l'une quelconque des revendications 1 à 4, dans laquelle la préparation est administrée par voie orale en une dose de 0,01 à 0,5 g/kg par jour.

6. Utilisation selon l'une quelconque des revendications 1 à 5, dans laquelle la préparation contient en outre des ions fers (Fe) ou d'autres ions de métaux bivalents qui sont des micro-éléments alimentaires en une dose de 0,0001 à 0,01 g/kg/jour.

7. Utilisation d'un alpha-céto-glutarate ou/et d'une glutamine ou/et d'un glutamate ou/et d'une omithine d'alpha-céto-glutarate, d'un dipeptide de glutamine et d'autres acides aminés ou/et de tripeptides de glutamine et d'autres acides aminés ou/et d'un glutamate avec un autre acide aminé ou/et des sels d'alpha-céto-glutarate ou/et d'une glutamine ou/et d'un glutamate ou/et d'une omithine d'alpha-céto-glutarate, de dipeptides de glutamine ou de glutamate avec un autre acide aminé pour la fabrication d'une préparation de diagnostic à utiliser pour augmenter l'absorption de manganèse (Mn) par la bile et le foie permettant l'utilisation de cette préparation en tant que composant du contraste oral utilisé pendant l'examen et le diagnostic des néoplasmes dans le foie et d'autres tissus du tube digestif chez les personnes et les animaux.
